# NEUE EUROPÄISCHE PATENTSCHRIFT

(11) **EP 0 625 355 B2**
(45) Veröffentlichungstag und Bekanntmachung des Hinweises auf die Entscheidung über den Einspruch: **25.06.2003**
(45) Hinweis auf die Patenterteilung: 27.09.1995
(21) Anmeldenummer: 93111827.7
(22) Anmeldetag: 24.07.1993
(51) Int. Cl.: A61K 47/38, A61K 9/20, A61K 31/66

(54) **Tablette mit verbesserter Bioverfügbarkeit enthaltend Dichlormethylendiphosphonsäure als Wirkstoff**
Tablet with improved bioavailability containing dichloromethylenedephosphonic acid as active ingredient
Comprimé à biodisponibilité améliorée contenant l'acide dichlorométhylène-diphosphonique en tant qu'ingrédient actif

(30) Priorität: 15.05.1993 DE 9307393 U; 02.07.1993 DE 4322057
(43) Veröffentlichungstag der Anmeldung: 23.11.1994
(73) Patentinhaber: Roche Diagnostics GmbH, 68298 Mannheim (DE)
(72) Erfinder: Preis, Walter, D-67433 Neustadt (DE); Müsel, Bernd, D-67551 Worms (DE); Neugebauer, Günter, Dr., D-68309 Mannheim (DE); Gabel, Rolf-Dieter, Dr., D-68723 Schwetzingen (DE)
(74) Vertreter: Weiss, Wolfgang, Dipl.-Chem. Dr.

(56) Entgegenhaltungen:
- EP-A- 0 124 027
- EP-A- 0 275 468
- EP-A- 0 336 851
- EP-A- 0 637 236
- EP-B- 0 479 813
- WO-A-93/21907
- Handbook of Pharmaceutical Excipients
- Bone, vol. 16, no. 2, 1995, pp. 275-279

## Beschreibung

Die Erfindung betrifft Tabletten mit verbesserter Bioverfügbarkeit des Wirkstoffes Dichlormethylendiphosphonsäure (Clodronsäure) oder eines physiologisch verträglichen Salzes davon und einem Zusatz an mikrokristalliner Cellulose als pharmazeutischem Hilfsstoff, Arzneimittelpackungen enthaltend diese Tabletten, die Verwendung von Dichlormethylendiphosphonsäure zusammen mit mikrokristalliner Cellulose zur Herstellung einer Tablette mit verbesserter Bioverfügbarkeit sowie das Verfahren zur Herstellung dieser Tablette.

Es ist bekannt, daß der Wirkstoff Dichlormethylendiphosphonsäure, auch bekannt unter dem Namen Clodronat, in Arzneimitteln zur Behandlung der Osteolyse infolge von Knochenmetastasen solider Tumoren sowie zur Behandlung der Hypercalcämie verwendet wird (vgl. z. B. DE-18 13 659). Inzwischen hat sich auch gezeigt, daß Biphosphonate wie z. B. Clodronsäure oder deren physiologisch verträgliche Salze bei der Behandlung von Osteoporose und Osteoporoseschmerz erfolgreich eingesetzt werden können.

Die Verbindung muß zur Behandlung der Osteolyse in relativ hoher Dosierung über lange Zeit appliziert werden, um ihre Wirkung zu entfalten. Das Präparat Ostac ^{(R)}-Kapseln enthält den Wirkstoff in Form seines Natriumsalzes (Clodronsäure, Dinatriumsalz x 4 H₂O; MW = 360 g/Mol) in einer Menge von 500 mg pro Kapsel. Bezogen auf den Wirkstoff Clodronsäure (MW = 244.9 g/Mol) entspricht dies einer Menge von etwa 340 mg. Für die Behandlung ist die Einnahme von vier Kapseln täglich, in schweren Fällen bis zu acht Kapseln pro Tag, erforderlich. Dies entspricht einer täglich zu verabreichenden Dosis von 1360 - 2720 mg Clodronsäure.

Aufgrund dieser erforderlichen hohen Dosierung des Wirkstoffes wurden zunächst Darreichungsformen entwickelt, die den Wirkstoff mit einem möglichst hohen Gehalt enthalten, um die Größe der einzelnen Darreichungsform möglichst klein zu gestalten. In EP 0 275 468 werden derartige Rezepturen mit einem Wirkstoffgehalt von 80 - 95 % beschrieben. Das Präparat Ostac ^{(R)} besitzt einen prozentualen Gehalt des Wirkstoffes Dinatriumclodronattetrahydrat von etwa 500 mg (entsprechend 91 %) bei einem Gesamtgewicht der Kapselfüllmasse von etwa 550 mg.

Für einige Patienten ist es aufgrund ihres Gesundheitszustandes problematisch, Kapseln einer solchen Größe zwischen 550 - 570 mg Füllmasse mehrmals täglich über einen längeren Zeitraum hinweg zu schlucken.

Andererseits wäre es aus Gründen der besseren Befolgung des Einnahmemodus durch die Patienten wünschenswert, die tägliche Einnahme von vier bis acht Kapseln zu reduzieren, da die Erfahrung zeigt, daß z. B. eine ein- oder zweimalige Einnahme konsequenter befolgt wird als eine mehrfache Applikation. Im Hinblick auf die erforderliche Gesamtdosis von etwa 1400 mg, in schweren Fällen von etwa 2700 mg Clodronat, die täglich verabreicht werden muß, wären damit zwei verhältnismäßig große Kapseln mit einem Gesamtgewicht von je mindestens 1100 mg erforderlich. Derartige Kapseln sind jedoch aufgrund ihrer Größe nachteilig.

Aufgabe der Erfindung war es, eine Darreichungsform mit erhöhter Bioverfügbarkeit des Wirkstoffes Clodronsäure zur Verfügung zu stellen, wodurch die täglich dem Patienten zu verabreichende Gesamtdosis reduziert und somit auch die Anzahl der täglich einzunehmenden Darreichungsformen erniedrigt werden kann bzw. bei gleicher Einnahmehäufigkeit kleinere Darreichungsformen, wie beispielsweise Tabletten, verwendet werden können.

Überraschenderweise wurde gefunden, daß als Darreichungsformen Tabletten mit einem Zusatz an mikrokristalliner Cellulose eine höhere Bioverfügbarkeit des Wirkstoffes im Vergleich mit den Kapseln bei der Applikation am Menschen bewirken. Dadurch ist es möglich, die zu applizierende Tagesdosis von Clodronsäure auf niedrigere Werte zu senken. Insbesondere wurde gefunden, daß die erfindungsgemäßen Tabletten eine Reduzierung der täglichen Gesamtdosis von Clodronsäure auf Werte bis zu 60 % ermöglichen. Dies bedeutet, daß beispielsweise anstelle der üblichen Menge von 1360 mg Clodronsäure eine tägliche Gesamtdosis von etwa 820 mg Clodronsäure einen vergleichbaren therapeutischen Effekt bewirkt.

Unter der Voraussetzung, daß ein Patient im Normalfall vier Kapseln des Präparates Ostac ^{(R)} mit einer Einzeldosis von etwa 340 mg Clodronsäure einnehmen mußte (entsprechend einer täglichen Gesamtdosis von 1360 mg Clodronsäure), führen die erfindungsgemäßen Tabletten zu einer Reduzierung der täglichen Gesamtdosis auf etwa 800 - 1100 mg. Dies bedeutet einerseits eine Reduzierung des Wirkstoffgehaltes pro Einzeldosierung auf etwa 200 - 270 mg Clodronsäure und Reduzierung des Gesamtgewichts der Tablette auf etwa 350 - 500 mg, wenn die Einnahme von vier Tabletten pro Tag beibehalten werden soll. Dies ist insbesondere für solche Patienten von Bedeutung, die Schwierigkeiten beim Schlucken von größeren Tabletten haben. Durch die Reduzierung des Wirkstoffgehaltes ergibt sich die Möglichkeit der Reduzierung des Gesamtgewichtes der Tablette, so daß relativ kleine Tabletten resultieren.

Um eine Reduzierung der Einnahmehäufigkeit zu erzielen, können andererseits Tabletten hergestellt werden. die die bisher übliche Einnahme von vier Ostac^{(R)}-Kapseln ersetzen. Durch die mit der erfindungsgemäßen Rezeptur erzielte hervorragende Bioverfügbarkeit ist es möglich, in Abhängigkeit von der gewünschten täglichen Einnahmehäufigkeit den Wirkstoffgehalt pro Tablette und somit die Tablettengröße in bestimmten Grenzen beliebig zu variieren.

Geht man beispielsweise von einer täglichen Gesamtdosis von 1360 mg Ciodronsäure aus, die bisher durch vier Ostac^{(R)}-Kapseln verabreicht wurden, und legt man die in Humanversuchen gefundene höhere Bioverfügbarkeit des Wirkstoffes in den erfindungsgemäßen Tabletten zugrunde, die eine Reduzierung der Gesamtdosis von Clodronsäure auf etwa 65 % (entsprechend 884 mg Clodronsäure) ermöglicht. so kann diese Tagesdosis durch zwei. drei oder vier erfindungsgemäße Tabletten mit einem Wirkstoffgehalt von je 442 mg, 295 mg oder 221 mg Clodronsäure verabreicht werden. Die Tablettengröße läßt sich somit an die jeweiligen Erfordernisse beliebig anpassen. Die größeren Tabletten sind insbesondere dann vorteilhaft. wenn zu befürchten ist, daß der Patient die mehrmals täglich erforderliche Einnahme nicht konsequent befolgt, und eine ein- oder zweimalige Einnahme pro Tag bevorzugt. Die kleineren Tabletten sind in solchen Fällen vorteilhaft, wenn der Patient Schwierigkeiten beim Schlucken der größeren Tabletten hat, und somit die Einnahme von kleineren Tabletten mehrmals am Tag bevorzugt. Bei einer höheren oder niedrigeren täglichen Gesamtdosis als der hier beispielhaft beschriebenen Dosis von 1360 mg Clodronsäure erfolgt die Festlegung des Wirkstoffgehaltes pro Tablette in analoger Weise, je nach den gewünschten Erfordernissen

hinsichtlich Einnahmehäufigkeit und Größe der Tabletten. Die folgenden Beispiele erläutern einige bevorzugte Ausführungsformen der Erfindung:
a) Eine übliche Ostac^{(R)}-Kapsel (340 mg Clodronsäure) kann durch eine erfindungsgemäße Tablette mit einem Wirkstoffgehalt von etwa 220 mg ersetzt werden. Für den Fall. daß der Wirkstoff in Form des Tetrahydrates des Natriumsalzes eingesetzt wird, bedeutet dies eine Reduktion der Wirkstoffmenge in der Darreichungsform von 500 mg auf etwa 325 mg.
b) Zwei übliche Ostac^{(R)}-Kapseln (Gesamtdosis 680 mg Clodronsäure) können durch eine erfindungsgemäße Tablette mit einem Wirkstoffgehalt von etwa 440 mg ersetzt werden. Dies bedeutet entsprechend den Angaben unter Punkt a) eine Verminderung der Wirkstoffmenge von 1000 mg auf 650 mg des Tetrahydrates von Dinatriumclodronat, bzw. bezogen auf die wasserfreie Form von 800 mg auf 520 mg.
c) Drei übliche Ostac^{(R)}-Kapseln (Gesamtdosis 1020 mg Clodronsäure) können durch eine erfindungsgemäße Tablette mit einem Wirkstoffgehalt von etwa 660 mg bzw. durch zwei Tabletten mit je 330 mg ersetzt werden.
d) Vier übliche Ostac^{(R)}-Kapseln (Gesamtdosis 1360 mg Clodronsäure) können durch eine erfindungsgemäße Tablette mit einem Wirkstoffgehalt von etwa 880 mg, bzw. durch zwei Tabletten mit je etwa 440 mg. bzw. drei Tabletten mit je etwa 300 mg Ciodronsäure ersetzt werden

Bei der obigen Berechnung des Wirkstoffgehaltes der erfindungsgemäßen Tablette wurden die Ergebnisse aus Bioäquivalenzstudien am Menschen zugrunde gelegt. aus denen hervorging, daß die Einnahme einer Tablette mit einem Wirkstoffgehalt von 520 mg (bezogen auf die wasserfreie Form des Natriumclodronats) bioäquivalent ist zur Einnahme von zwei üblichen Ostac^{(R)}-Kapseln mit jeweils 400 mg Wirkstoffgehalt. Die Messung der Serumkonzentration von Clodronsäure im Blut von mehreren Patienten ergab weitgehend vergleichbare Werte über einen Zeitraum von 16 Stunden.

Die erfindungsgemäßen Tabletten können insbesondere zur Behandlung der Osteoporose eingesetzt werden. Da es sich in diesen Fällen häufig um eine Langzeittherapie handelt, ist die vorteilhafte Reduzierung der Tagesdosis von Clodronsäure und somit die Minimierung von möglichen Nebenwirkungen von besonderer Bedeutung.

Die erfindungsgemäße Tablette enthält den Wirkstoff Dichlormethylendiphosphonsäure in einer Menge von 200 - 700 mg, bezogen auf den Gehalt von Clodronsäure. Bevorzugt werden die physiologisch verträglichen Salze der Clodronsäure, insbesondere die Alkalisalze, bevorzugt das Dinatriumsalz eingesetzt. das entweder als Tetrahydrat oder in wasserfreier Form verwendet werden kann. Selbstverständlich können auch andere physiologisch verträgliche Salze. wie z. B. das Lithium-, Kalium-, Ammonium- oder Caiciumsalz bzw. deren Hydrate eingesetzt werden. Der prozentuale Gehalt des Wirkstoffes (bezogen auf Clodronsäure) beträgt 10 - 65 Gew.-%, bevorzugt 50 - 60 Gew.-%. insbesondere etwa 55 Gew.-%. bezogen auf das Gesamtgewicht des Tablettenkerns. Bei Verwendung des Natriumclodronattetrahydrates als Bezugsgröße beträgt die Wirkstoffmenge bevorzugt 74 - 88 %. insbesondere etwa 80 % des Gesamtgewichts der Tablette.

Der erfindungsgemäße Zusatz von mikrokristalliner Cellulose beträgt bezogen auf das Gesamtgewichts des Tablettenkerns 5 - 15 Gew.-%, insbesondere 8 - 12 Gew.-%. Besonders bevorzugt beträgt der Gehalt an mikrokristalliner Cellulose etwa 10 Gew.-%. Die mikrokristalline Cellulose wird vorzugsweise als Avicel^{(R)} eingesetzt. Anstelle der mikrokristalliner Cellulose können auch andere gleichwirkende Mittel, wie z.B. andere modifizierte Cellulose-Derivate oder Polyethylenglykol (PEG) 4000 - 6000 eingesetzt werden.

Die Tablette enthält weiterhin einen oder mehrere pharmazeutisch übliche Hilfs- oder Trägerstoffe, wie z.B. Füllstoffe, Gleitmittel, Sprengmittel, Bindemittel oder Formentrennmittel. Als solche kommen Stärke (Kartoffel-, Weizen-und Maisstärke), Lactose, Glucose, Mannit, Calciumcarbonat, Calciumphosphat, Cellulose, Talkum oder andere für diesen Zweck in der Technik bekannte Produkte in Frage. Der Anteil der pharmazeutischen Hilfs- und Trägerstoffe kann in Abhängigkeit von dem gewählten Wirkstoffgehalt der Tablette in weiten Grenzen variieren und beträgt jeweils 0,1 - 20 Gew.-%.

Der Anteil der Füllstoffe beträgt etwa 3 - 10 %, vorzugsweise 5 - 7 Gew.-%, bezogen auf das Gesamtgewicht der Tablette. Als Füllstoffe kommen insbesondere Maisstärke, Talkum und/oder Lactose in Frage. Der Anteil von Talkum beträgt bevorzugt etwa 3,5 - 5 %, der Anteil von Maisstärke etwa 2 - 5 %, insbesondere etwa 2,5 Gew.-%.

Die Tablette kann übliche Gleitmittel enthalten. Als solche kommen bevorzugt Siliciumdioxid, Talkum und/oder Stearinsäure oder deren Salze in Frage, insbesondere deren Magnesium- oder Calciumsalze. Der Gesamtgehalt an Gleitmitteln beträgt bis zu 6 Gew.-% bezogen auf das Gesamtgewicht der Tablette. Es können ein oder mehrere Gleitmittel in jeweils gleichen oder unterschiedlichen Mengen verwendet werden. Bevorzugt beträgt der Gehalt jeweils bis zu 3 Gew.-%, insbesondere 0,1 - 2 Gew.-%. Bevorzugt wird Magnesiumstearat und/oder Talkum im Bereich von jeweils 0,2 - 2 Gew.-% eingesetzt.

Die Tablette kann ferner außer den oben genannten Hilfsstoffen noch Tablettensprengmittel hinzugefügt werden, die einen rascheren Zerfall der Tablette beim Kontakt mit der Magenflüssigkeit bewirken. Derartige Sprengmittel sind beispielsweise Natriumcarboxymethylstärke, Crosscarmellose, Crospovidone und andere gleichwirkende Mittel, die in Mengen bis zu 10 Gew.-%, vorzugsweise bis zu 3 Gew.-%, bezogen auf das Gesamtgewicht der Tablette, vorhanden sein können. Vorzugsweise wird als Sprengmittel Natriumcarboxymethylstärke im Bereich von 1 - 5 Gew.-%, bevorzugt 1 - 2 Gew.-% eingesetzt.

Der Tablettenkern, der als Bezugsgröße für die Berechnung der oben genannten Gewichtsverhältnisse dient, kann mit einem Überzug versehen werden. Der Überzug kann einerseits dazu dienen, den unangenehmen Geschmack der Tablette als solche zu vermeiden. In diesem Fall werden bei der Herstellung des Tablettenüberzuges Geschmacksstoffe zugesetzt. Der Überzug kann andererseits auch die Freisetzung des Wirkstoffes verzögern. Hierzu werden Stoffe eingesetzt, die in Form eines aufgetragenen Diffusionsfilmes zu einer retardierten Freisetzung des Wirkstoffes beitragen.

In Bioverfügbarkeitsstudien zeigte sich, daß beispielsweise eine erfindungsgemäße Tablette mit einem Wirkstoffgehalt von etwa 420 - 460 mg an Clodronsäure eine Bioverfügbarkeit des Wirkstoffes aufweist, die der von zwei üblichen Kapseln mit je etwa 340 mg Clodronsäure entspricht. Das Gesamtgewicht dieser erfindungsgemäßen Tablette, die damit das Doppelte der üblichen Dosis enthält, beträgt bei Verwendung des Tetrahydrats des Natriumclodronats zwischen 750 - 850 mg, vorzugsweise 790 - 810 mg. Bei Verwendung des wasserfreien Clodronats resultiert durch die erforderliche geringere Einwaage des Wirkstoffes ein niedrigeres Gesamtgewicht der Tablette. In diesen Fällen kann es zweckmäßig sein, bei der Herstellung der Tablette den Anteil der pharmazeutischen Hilfs- und Trägerstoffe entsprechend auf die niedrigere Einwaage des Wirkstoffes im gleichen Verhältnis zu reduzieren.

Ebenso können nach der erfindungsgemäßen Rezeptur Tabletten mit einem Wirkstoffgehalt von 500 - 530 mg und einem maximalen Gesamtgewicht (bezogen auf Natriumclodronat-Tetrahydrat) von 870 - 970, vorzugsweise 900 - 950 mg, hergestellt werden. Damit können erfindungsgemäß Tabletten mit Einzel- und doppelten Dosen zur Verfügung gestellt werden, die kleiner und damit besser oral applizierbar sind als es die nach den bisher bekannten Rezepturen wären. Für eine bisher übliche Kapsel mit 340 mg Wirkstoffgehalt an Clodronsäure (entsprechend etwa 500 mg Dinatriumclodronattetrahydrat, bzw. 400 mg wasserfreiem Dinatriumclodronat) sind nach der erfindungsgemäßen Rezeptur nur noch ca. 220 mg Wirkstoff erforderlich, wodurch das Gesamtgewicht der Tablette in der Regel zwischen 395 - 410 mg liegt.

Die erfindungsgemäße Tablette zeigt auch ein gutes Auflösungsverhalten. So beträgt die Auflösungsrate der erfindungsgemäßen 440 mg-Dosierung (bestimmt nach der USP-paddle-method) bereits nach 15 Minuten mindestens 60 % und nach 30 Minuten mindestens 75 %.

Gegenstand der Erfindung sind auch Arzneimittelpackungen enthaltend 30 - 400 erfindungsgemäße Tabletten zur Verabreichung in einer täglichen Dosis von ein bis drei, vorzugsweise zwei Tabletten (Wirkstoffgehalt 420 - 460 bzw. 500 - 530 mg) oder vier bis acht, vorzugsweise vier, Tabletten (Wirkstoffgehalt 200 - 270 mg).

Gegenstand der Erfindung ist weiterhin die Verwendung des Wirkstoffes Dichlormethylendiphosphonsäure oder eines physiologisch verträglichen Salzes davon zusammen mit mikrokristalliner Cellulose als pharmazeutischem Hilfsstoff zur Herstellung einer Tablette mit verbesserter Bioverfügbarkeit, vorzugsweise einer Tablette üblicher Größe mit zweifacher Wirksamkeit gegenüber einer üblichen Kapsel.

Die erfindungsgemäßen Tabletten werden in üblicher Weise hergestellt, indem die Tablettenmasse vor der Verpressung durch Granulation (Trocken-, Feucht- oder Sprühgranulation) in eine geeignete gekörnte Form gebracht wird. In der Regel wird die gewünschte Menge des Wirkstoffes für die herzustellende Darreichungsform mit ca. 4 - 8 Gew.-% der Füllstoffe trocken gemischt und mit einem üblichen Bindemittel, beispielsweise Maisstärke, oder auch nur mit Wasser granuliert. Darüber hinaus können auch andere Verfahren wie das der Kompaktierung angewandt werden. Das so erhaltene Granulat wird dann in einer handelsüblichen Mischapparatur mit 5-15 Gew.% mikrokristalliner Cellulose, bis zu 6 Gew.% Gleitmittel und bis zu 3 Gew.% Sprengmittel versetzt und gemischt. Nach dem Mischvorgang wird das Granulat tablettiert oder gegebenenfalls vorher noch mit einer Aromalösung besprüht und zur Durchdringung gelagert. Zur Geschmacksverbesserung kann auch die fertige Tablette mit einem Film überzogen werden.

Die Dosierungsangaben in den folgenden Ausführungsbeispielen beziehen sich auf den Gehalt an Clodronsäure (MW = 244.9). Zur Umrechnung auf den Gehalt an Dinatriumclodronat, wasserfrei (MW = 288.9) beträgt der Umrechnungsfaktor etwa 1.18, im Fall des Tetrahydrates (MW = 360.9) etwa 1.47.

### Beispiel 1:

### Tabletten mit einem Wirkstoffgehalt von 440 mg Clodronsäure

a) Im folgenden wird die Herstellung einer Ansatzgröße von 200.000 Tabletten mit einem Wirkstoffgehalt von Clodronsäure von 440 mg (entsprechend 520 mg Natriumclodronat, wasserfrei; bzw. 650 mg Natriumclodronattetrahydrat) beschrieben.

| | | |
|---|---|---|
| Pos. 1 | Clodronsäure Dinatriumtetrahydrat | 129945,4 g |
| Pos. 2 | Maisstärke | 3900,0 g |
| Pos. 3 | Talkum | 5980,0 g |
| Pos. 4 | Natriumcarboxymethylstärke | 2654,6 g |
| Pos. 5 | Magnesiumstearat | 520,0 g |
| Pos. 6 | Mikrokristalline Cellulose | 15600,0 g |
| | Ansatzgewicht: | 158600,0 g |

Die Einsatzstoffe aus den Pos. 1-3 werden granuliert. Dem Granulat werden anschließend die Zusatzstoffe aus den Pos. 4-6 zugemischt. Die so hergestellte Masse wird anschließend auf geeigneten Maschinen zu Tabletten verpreßt. Die Ausbeute an optisch einwandfreien Tabletten beträgt 177.215 Stück (88.6 %).
b) Bei Verwendung der wasserfreien Form des Natriumclodronats ergibt sich analog zu Beispiel 1a) folgende Zusammensetzung der pharmazeutischen Mischung:

| | | |
|---|---|---|
| Pos. 1 | Clodronsäure Dinatriumsalz, wasserfrei | 104000,0 g |
| Pos. 2 | Maisstärke | 3121,3 g |
| Pos. 3 | Talkum | 4786,0 g |
| Pos. 4 | Natriumcarboxymethylstärke | 2124,6 g |
| Pos. 5 | Magnesiumstearat | 416,2 g |
| Pos. 6 | Mikrokristalline Cellulose | 12485,3 g |
| | Ansatzgewicht: | 126933,4 g |

Die Herstellung des Granulates und der Tablettenmasse erfolgt analog zu Beispiel 1a). Die folgende Tabelle gibt die Zusammensetzung des Tablettenkerns wieder:

| | 440 mg - Dosierung |
|---|---|
| Natriumclodronat x 4 H₂O | 649.727 |
| Talkum | 29.90 |
| Maisstärke | 19.50 |
| Mikrokrist.Cellulose | 78.00 |
| Natriumcarboxymethylstärke | 13.273 |
| Magnesiumstearat | 2.6 |
| Gewicht der Tablette / mg | 793 |

Die Tabletten eignen sich insbesondere für eine Applikation von 2 Tabletten pro Tag und ersetzen damit 4 herkömmliche Ostac-Kapseln.

### Beispiel 2

### Tabletten mit einem Wirkstoffgehalt von 509 mg Clodronsäure

Die Herstellung erfolgt analog zu Beispiel 1 für eine Ansatzgröße von 200.000 Tabletten mit einem Wirkstoffgehalt von Clodronsäure von 509 mg (entsprechend 600 mg Natriumclodronat, wasserfrei; bzw. 750 mg Natriumclodronattetrahydrat) pro Tablette.

| | | |
|---|---|---|
| Pos. 1 | Clodronsäure Dinatriumtetrahydrat | 150000 g |
| Pos. 2 | Maisstärke | 4500 g |
| Pos. 3 | Talkum | 6900 g |
| Pos. 4 | Natriumcarboxymethylstärke | 3063 g |
| Pos. 5 | Magnesiumstearat | 600 g |
| Pos. 6 | Mikrokristalline Cellulose | 18000 g |
| | Ansatzgewicht: | 183063 g |

Die folgende Tabelle gibt die Zusammensetzung pro Tablettenkerns wieder:

| | 509 mg - Dosierung |
|---|---|
| Natriumclodronat x 4 H₂O | 749,685 mg |
| Talkum | 34,50 mg |
| Maisstärke | 22,50 mg |
| Mikrokrist.Cellulose | 90.00 mg |
| Natriumcarboxymethylstärke | 15.315 mg |
| Magnesiumstearat | 3,0 mg |
| Gewicht der Tablette / mg | 915 mg |

Die Tabletten eignen sich insbesondere für eine Applikation von 2 Tabletten pro Tag und ersetzen damit 4 herkömmliche Ostac-Kapseln.

### Beispiel 3

### Tablette mit einem Wirkstoffgehalt von 678 mg Clodronsäure

Die Herstellung erfolgt analog zu Beispiel 1 für eine Ansatzgröße von 100.000 Tabletten mit einem Wirkstoffgehalt von Clodronsäure von 678 mg (entsprechend 800 mg Natriumclodronat, wasserfrei; bzw. 1000 mg Natriumclodronattetrahydrat) pro Tablette.

| | | |
|---|---|---|
| Pos. 1 | Clodronsäure Dinatriumtetrahydrat | 99958 g |
| Pos. 2 | Maisstärke | 3000 g |
| Pos. 3 | Talkum | 4600 g |
| Pos. 4 | Natriumcarboxymethylstärke | 2042 g |
| Pos. 5 | Magnesiumstearat | 400 g |
| Pos. 6 | Mikrokristalline Cellulose | 12000 g |
| | Ansatzgewicht: | 122000,0 g |

Die folgende Tabelle gibt die Zusammensetzung pro Tablettenkerns wieder:

| | **678 mg - Dosierung** |
|---|---|
| Natriumclodronat x 4 H₂O | 10000,0 |
| Talkum | 46,0 |
| Maisstärke | 30,0 |
| Mikrokrist.Cellulose | 120,0 |
| Natriumcarboxymethylstärke | 20,42 |
| Magnesiumstearat | 4,0 |
| Gewicht der Tablette / mg | 1220 |

Die vorstehende Tablette ersetzt ca. drei herkömmliche Ostac^{(R)}-Kapseln.

### Beispiel 4

### Tablette mit einem Wirkstoffgehalt von 220 mg Clodronsäure

Die Herstellung erfolgt analog zu Beispiel 1 für eine Ansatzgröße von 300.000 Tabletten mit einem Wirkstoffgehalt von Clodronsäure von 220 mg (entsprechend 260 mg Natriumclodronat, wasserfrei; bzw. 325 mg Natriumclodronattetrahydrat) pro Tablette.

| | | |
|---|---|---|
| Pos. 1 | Clodronsäure Dinatriumtetrahydrat | 97459 g |
| Pos. 2 | Maisstärke | 2925 g |
| Pos. 3 | Talkum | 4485 g |
| Pos. 4 | Natriumcarboxymethylstärke | 1992 g |
| Pos. 5 | Magnesiumstearat | 390 g |
| Pos. 6 | Mikrokristalline Cellulose | 11700 g |
| | Ansatzgewicht: | 118951 g |

Die folgende Tabelle gibt die Zusammensetzung pro Tablettenkerns wieder:

| | 220 mg Dosierung |
|---|---|
| Natriumclodronat x 4 H₂O | 324,864 |
| Talkum | 14,95 |
| Maisstärke | 9,75 |
| Mikrokrist.Cellulose | 39,0 |
| Natriumcarboxymethylstärke | 6,64 |
| Magnesiumstearat | 1,30 |
| Gewicht der Tablette / mg | 396,5 |

Die Tabletten eignen sich insbesondere für eine Verabreichung von 4 Tabletten pro Tag, in schweren Fällen von 8 Tabletten pro Tag. Sie ersetzen die herkömmlichen Ostac^{(R)}-Kapseln, deren Gesamtgewicht bei Verwendung des Natriumchlodronat-Tetrahydrats 550 mg beträgt.

### Beispiel 5

### Tablette mit einem Wirkstoffgehalt von 254 mg Clodronsäure

Die Herstellung erfolgt analog zu Beispiel 1 für eine Ansatzgröße von 300.000 Tabletten mit einem Wirkstoffgehalt von Clodronsäure von 254 mg (entsprechend 300 mg Natriumclodronat, wasserfrei; bzw. 375 mg Natriumclodronattetrahydrat) pro Tablette.

| | | |
|---|---|---|
| Pos. 1 | Clodronsäure Dinatriumtetrahydrat | 112452 g |
| Pos. 2 | Maisstärke | 3375 g |
| Pos. 3 | Talkum | 5175 g |
| Pos. 4 | Natriumcarboxymethylstärke | 2298 g |
| Pos. 5 | Magnesiumstearat | 450 g |
| Pos. 6 | Mikrokristalline Cellulose | 13500 g |
| | Ansatzgewicht: | 137337 g |

Die folgende Tabelle gibt die Zusammensetzung pro Tablettenkerns wieder:

| | 254 mg Dosierung |
|---|---|
| Natriumclodronat x 4 H₂O | 374,84 |
| Talkum | 17,25 |
| Maisstärke | 11,25 |
| Mikrokrist.Cellulose | 45,0 |
| Natriumcarboxymethylstärke | 7,66 |
| Magnesiumstearat | 1,50 |
| Gewicht der Tablette / mg | 457,5 |

Die Tabletten eignen sich insbesondere für eine Verabreichung von 4 Tabletten pro Tag, in schweren Fällen von 8 Tabletten pro Tag. Sie ersetzen die herkömmlichen Ostac^{(R)}-Kapseln, deren Gesamtgewicht bei Verwendung des Natriumchlodronat-Tetrahydrats 550 mg beträgt.

### Beispiel 6

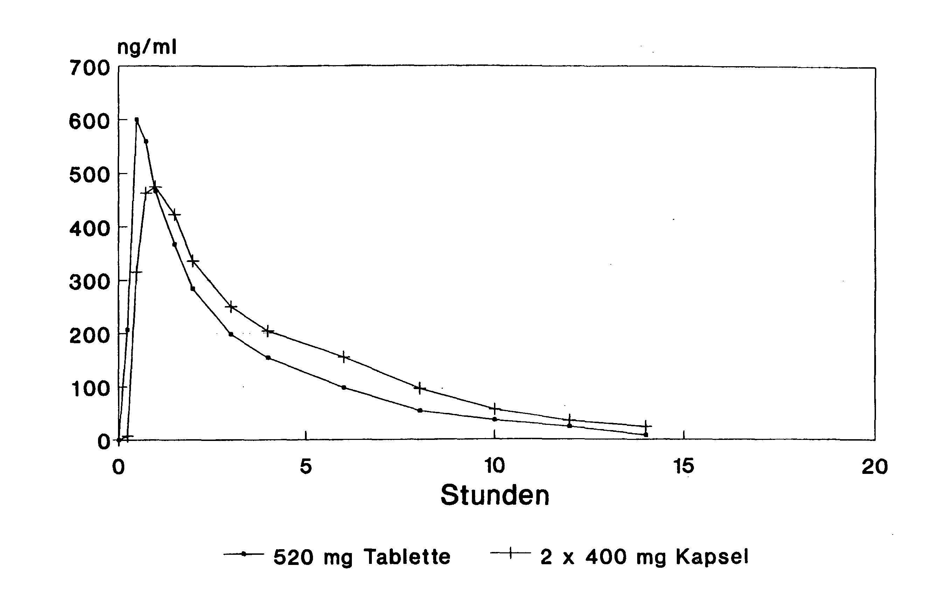

24 Patienten erhielten die übliche Dosierung von zwei Ostac^{(R)}-Kapseln mit einem Wirkstoffgehalt von je 340 mg Clodronsäure. Die Serumkonzentration von Clodronsäure wurde nach Standardmethoden über einen Zeitraum von 16 Stunden gemessen. Der zeitliche Verlauf der Serumkonzentration geht aus Abb. 1 (Kurve +) hervor.

Die gleiche Patientengruppe erhielt eine Tablette mit einem Wirkstoffgehalt von 440 mg Clodronsäure. Im Vergleich zu der mit Ostac^{(R)} behandelten Gruppe ist der Verlauf der mittleren Serumkonzentration von Clodronsäure weitgehend identisch, d.h. bioäquivalent bezüglich der Verabreichung von zwei Ostac^{(R)}-Kapseln.

## Patentansprüche

1. Tablette mit verbesserter Biovergübarkeit des Wirkstoffes Clodronsäure oder eines physiologisch verträglichen Salzes davon, einem Gehalt von 5 - 15 % (bezogen auf das Gesamtgewicht der Tablette) mikrokristalliner Cellulose als pharmazeutischem Hilfsstoff und einem Wirkstoffgehalt an Clodronsäure von 200 - 700 mg.

2. Tablette nach Anspruch 1 mit einem Gehalt an mikrokristalliner Cellulose von 8 - 12 %.

3. Tablette nach den Ansprüchen 1 oder 2, enthaltend den Wirkstoff in Form des Dinatriumsalzes, vorzugsweise in Form des Tetrahydrates.

4. Tablette nach den Ansprüchen 1 - 3, zusätzlich enthaltend einen Füllstoff, insbesondere Maisstärke oder Talkum.

5. Tablette nach den Ansprüchen 1 - 4, zusätzlich enthaltend ein Gleitmittel und ein Sprengmittel.

6. Tablette nach Anspruch 5, wobei das Gleitmittel ein physiologisch verträgliches Salz der Stearinsäure, vorzugsweise Magnesiumstearat, und das Sprengmittel Natriumcarboxymethylstärke.

7. Tablette nach den Ansprüchen 1 - 6 mit einem Wirkstoffgehalt an Clodronsäure von 420 - 460 mg und einem Gesamtgewicht der Tablette von 750 - 850 mg, vorzugsweise 790 - 810 mg.

8. Tablette nach den Ansprüchen 1 - 6 mit einem Wirkstoffgehalt an Clodronsäure von 500 - 530 mg und einem Gesamtgewicht der Tablette von 870 - 970 mg, vorzugsweise 900 - 950 mg.

9. Tablette nach den Ansprüchen 1 - 6 mit einem Wirkstoffgehalt an Clodronsäure von etwa 200 - 270 mg und einem Gesamtgewicht der Tablette von 350 - 500 mg.

10. Arzneimittelpackung, enthaltend 30 - 400 Tabletten gemäß der Ansprüche 7 oder 8 zur Verabreichung in einer täglichen Dosis von ein bis drei Tabletten, vorzugsweise zwei Tabletten.

11. Arzneimittelpackung, enthaltend 30 - 400 Tabletten gemäß Anspruch 9 zur Verabreichung in einer täglichen Dosis von vier bis acht Tabletten, vorzugsweise vier Tabletten.

12. Verwendung von mikrokristalliner Cellulose in einer Menge ven 5-15% (bezogen auf das gesamtgewicht der Tablette) als pharmazeutischem Hilfsstoff zur Erhöhung der Bioverfügbarkeit des Wirkstoffes Clodronsäure oder eines physiologisch verträglichen Salzes davon bei der Hersteilung von Tabletten.

13. Verfahren zur Herstellung einer Tablette nach den Ansprüchen 1 - 9, **dadurch gekennzeichnet, daß** man den Wirkstoff zusammen mit mikrokristalliner Cellulose und gegebenenfalls anderen pharmazeutischen Hilfs- oder Trägerstoffen nach an sich bekannten Verfahren mischt und zu Tabletten verpreßt.

14. Verfahren nach Anspruch 13, **dadurch gekennzeichnet, daß** man den Wirkstoff mit ca. 4 - 8 Gew.-% der Füllstoffe mischt, mit einem üblichen Bindemittel granuliert, dem so erhaltenen Granulat in einer üblichen Mischapparatur 5 - 15 Gew.-% mikrokristalline Cellulose, bis zu 3 Gew.-% Sprengmittel und bis zu 6 Gew.-% Gleitmittel zumischt, das Granulat nach dem Mischvorgang tablettiert und gegebenenfalls den erhaltenen Tablettenkern mit einem Überzug zur Geschmacksverbesserung oder zur retardierten Freisetzung des Wirkstoffes versieht.

15. Verfahren nach Anspruch 14, **dadurch gekennzeichnet, daß** man den Wirkstoff mit 6 - 7 Gew.-% der Füllstoffe granuliert und dem Granulat 8 - 12 Gew.-% mikrokristalline Cellulose, bis zu 2 Gew.-% Sprengmittel und bis zu 3 Gew.-% Gleitmittel zumischt.

16. Verfahren nach einem der Ansprüche 14 oder 15, **dadurch gekennzeichnet, daß** als Füllstoffe Maisstärke und/oder Talkum eingesetzt werden.

17. Verfahren nach einem der Ansprüche 14 - 16, **dadurch gekennzeichnet, daß** als Gleitmittel Magnesiumstearat und als Sprengmittel Natriumcarboxymethylstärke eingesetzt werden.

## Claims

1. Tablet with improved bioavailability of the active ingredient clodronic acid or a physiologically acceptable salt thereof, a content of 5 - 15% (based on the total weight of the tablet) of microcrystalline cellulose as a pharmaceutical adjunct, and a content of clodronic acid active ingredient of 200 - 700 mg.

2. Tablet according to Claim 1 with a microcrystalline cellulose content of 8 - 12%.

3. Tablet according to Claim 1 or 2 containing the active ingredient in the form of the disodium salt, preferably in the form of the tetrahydrate.

4. Tablet according to Claims 1 - 3 additionally containing a filler, especially maize starch or talcum.

5. Tablet according to Claims 1 - 4 additionally containing a lubricant and a disintegrating agent.

6. Tablet according to Claim 5 wherein the lubricant a physiologically acceptable salt of stearic acid, preferably magnesium stearate, and the disintegrating agent sodium carboxymethyl starch.

7. Tablet according to Claims 1 - 6 with a clodronic acid active ingredient content of 420 - 460 mg and a total tablet weight of 750 - 850 mg, preferably of 790 - 810 mg.

8. Tablet according to Claims 1 - 6 with a clodronic acid active ingredient content of 500 - 530 mg and a total tablet weight of 870 - 970 mg, preferably of 900 - 950 mg.

9. Tablet according to Claims 1 - 6 with a clodronic acid active ingredient content of about 200 - 270 mg and a total tablet weight of 350 - 500 mg.

10. Drug pack containing 30 - 400 tablets according to Claim 7 or 8 for administration in a daily dose of one to three tablets, preferably of two tablets.

11. Drug pack containing 30 - 400 tablets according to Claim 9 for administration in a daily dose of four to eight tablets, preferably of four tablets.

12. Use of microcrystalline cellulose in an amount of 5 - 15% (based on the total weight of the tablet) as a pharmaceutical adjunct for increasing the bioavailability of the active ingredient clodronic acid or a physiologically acceptable salt thereof in the production of tablets.

13. Process for the production of a tablet according to Claims 1 - 9, **characterized in that** the active ingredient is mixed together with microcrystalline cellulose and optionally other pharmaceutical adjuncts or excipients, by methods known per se, and compressed to tablets.

14. Process according to Claim 13, **characterized in that** the active ingredient is mixed with approx. 4 - 8 wt.% of fillers and granulated with a conventional binder, 5 - 15 wt.% of microcrystalline cellulose, up to 3 wt.% of disintegrating agent and up to 6 wt.% of lubricant are admixed to the resulting granules in a conventional mixing apparatus, the granules are converted to tablets after the mixing process and the tablet core obtained is optionally provided with a coating to improve the taste or to delay the release of the active ingredient.

15. Process according to Claim 14, **characterized in that** the active ingredient is granulated with 6 - 7 wt.% of fillers, and 8 - 12 wt.% of microcrystalline cellulose, up to 2 wt.% of disintegrating agent and up to 3 wt.% of lubricant are admixed to the granules.

16. Process according to Claim 14 or 15, **characterized in that** the fillers used are maize starch and/or talcum.

17. Process according to one of Claims 14 - 16, **characterized in that** the lubricant used is magnesium stearate and the disintegrating agent used is sodium carboxymethyl starch.

## Revendications

1. Comprimé possédant une biodisponibilité améliorée du principe actif, l'acide clodronique ou l'un de ses sels physiologiquement acceptables ayant une teneur en cellulose microcristalline allant de 5% à 15% (par rapport au poids total du comprimé) en tant qu'adjuvant pharmaceutique et une teneur en principe actif, à savoir l'acide clodronique, allant de 200 mg à 700 mg.

2. Comprimé selon la revendication 1, ayant une teneur en cellulose microcristalline allant de 8% à 12%.

3. Comprimé selon la revendication 1 ou 2, contenant le principe actif sous la forme de son sel disodique, de préférence sous la forme de son tétrahydrate.

4. Comprimé selon les revendications 1 à 3, contenant en plus une charge, en particulier de l'amidon de maïs ou du talc.

5. Comprimé selon les revendications 1 à 4, contenant en plus un lubrifiant et un délitant.

6. Comprimé selon la revendication 5, dans lequel le lubrifiant est un sel, physiologiquement acceptable, de l'acide stéarique, de préférence le stéarate de magnésium, et le délitant est le carboxyméthylamidon sodique.

7. Comprimé selon les revendications 1 à 6, ayant une teneur en principe actif, à savoir l'acide clodronique, allant de 420 mg à 460 mg et un poids total du comprimé allant de 750 mg à 850 mg, de préférence de 790 mg à 810 mg.

8. Comprimé selon les revendications 1 à 6, ayant une teneur en principe actif, à savoir l'acide clodronique, allant de 500 mg à 530 mg et un poids total du comprimé allant de 870 mg à 970 mg, de préférence de 900 mg à 950 mg.

9. Comprimé selon les revendications 1 à 6, ayant une teneur en principe actif, à savoir l'acide clodronique, d'environ 200 mg à 270 mg et un poids total du comprimé allant de 350 mg à 500 mg.

10. Conditionnement de médicaments contenant de 30 à 400 comprimés selon les revendications 7 ou 8 pour l'administration d'une dose journalière de un à trois comprimés, de préférence de deux comprimés.

11. Conditionnement de médicaments contenant de 30 à 400 comprimés selon la revendication 9 pour l'administration d'une dose journalière de quatre à huit comprimés, de préférence de quatre comprimés.

12. Utilisation de cellulose microcristalline, à raison de 5% à 15% (par rapport au poids total du comprimé), en tant qu'adjuvant pharmaceutique en vue d'augmenter la biodisponibilité du principe actif, à savoir l'acide clodronique ou l'un de ses sels physiologiquement acceptables, lors de la fabrication de comprimés.

13. Procédé pour la fabrication d'un comprimé selon les revendications 1 à 9, **caractérisé en ce que** l'on mélange, selon des procédés connus en soi, le principe actif conjointement avec la cellulose microcristalline et éventuellement d'autres adjuvants ou excipients pharmaceutiques et qu'on comprime le mélange pour former des comprimés.

14. Procédé selon la revendication 13, **caractérisé en ce que** l'on mélange le principe actif avec environ 4% à 8% en poids de charge, que l'on granule ce mélange à l'aide d'un liant classique, que l'on mélange au granulé ainsi obtenu, dans un mélangeur classique, 5% à 15% en poids de cellulose microcristalline, jusqu'à 3% en poids de délitant et jusqu'à 6% en poids de lubrifiant, que l'on forme le granulé en comprimé après l'étape de mélange et, éventuellement, que l'on enrobe le noyau des comprimés obtenus pour obtenir une amélioration du goût ou une libération prolongée du principe actif.

15. Procédé selon la revendication 14, **caractérisé en ce que** l'on granule le principe actif avec 6% à 7% en poids de charge et que l'on mélange au granulé 8% à 12% en poids de cellulose microcristalline, jusqu'à 2% en poids de délitant et jusqu'à 3% en poids de lubrifiant.

16. Procédé selon l'une quelconque des revendications 14 ou 15, **caractérisé en ce que** l'on met en oeuvre, en tant que charge, de l'amidon de maïs et/ou du talc.

17. Procédé selon l'une quelconque des revendications 14 à 16, **caractérisé en ce que** l'on met en oeuvre, en tant que lubrifiant, du stéarate de magnésium et, en tant que délitant, du carboxyméthylamidon sodique.
